# EUROPEAN PATENT APPLICATION

(11) **EP 4 534 085 A1**
(43) Date of publication of application: **09.04.2025**
(21) Application number: 23201706.1
(22) Date of filing: 04.10.2023
(51) Int. Cl.: A61K 31/4427, A61P 35/00

(54) **COMPOUNDS FOR USE IN METHODS OF TREATING CANCER**

(30) Priority: 03.10.2023 US 202363542245 P
(71) Applicant: Scorpion Therapeutics, Inc., Boston, MA 02110 (US)
(72) Inventor: Guzman-Perez, Angel, Belmont, Massachusetts, 02478 (US); Milgram, Benjamin C., Weston, Massachusetts, 02493 (US); White, Ryan D., Somerville, Massachusetts, 02144 (US); St. Jean, Jr., David, Natick, Massachusetts, 02110 (US)
(74) Representative: Fish & Richardson P.C.

(57) **Abstract**

The present disclosure relates to the use of an EGFR inhibitor or a pharmaceutically acceptable salt thereof for treating a cancer having two or more mutations in EGFR, wherein at least one of the mutations is a C797 mutation; preferably the C797S mutation. The EGFR inhibitor disclosed herein is a compound of formula (I): 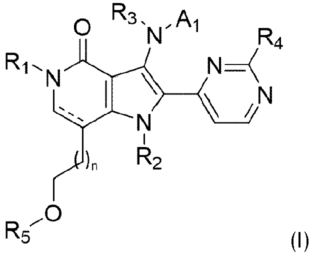 or a pharmaceutically acceptable salt or N-oxide or solvate thereof.

## Description

### TECHNICAL FIELD

The present disclosure relates to the use of an EGFR inhibitor or a pharmaceutically acceptable salt thereof for treating a cancer having two or more mutations in EGFR, wherein at least one of the mutations is a C797 mutation; preferably the C797S mutation. The EGFR inhibitor disclosed herein is a compound of formula (I): 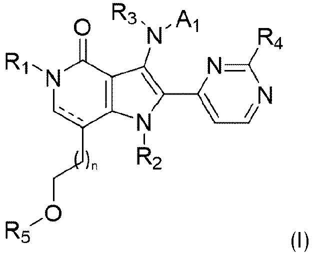 or a pharmaceutically acceptable salt or N-oxide or solvate thereof.

### BACKGROUND

Lung cancer is the leading cause of cancer deaths worldwide. Metastatic non-small- cell lung cancer (NSCLC) has recently benefited from two consecutive breakthroughs: the identification of oncogene drivers, such as EGFR mutations, leading to the development of targeted therapies, and the understanding of the cancer immunity cycle leading to the development of immune checkpoint inhibitors.

The epidermal growth factor receptor (EGFR) is overexpressed, dysregulated, or mutated in many epithelial malignancies, and EGFR activation appears important in tumour growth and progression. Activation of EGFR stimulates tumour growth and progression, including the promotion of proliferation, angiogenesis, invasion, metastasis, and inhibition of apoptosis.

EGFR mutations are well validated clinical targets in non-small cell lung cancer (NSCLC). Osimertinib, a highly-selective mutation targeting covalent drug, is increasingly used in the first line setting for patients with NSCLC bearing EGFR L858R mutation or exon 19 deletion (exdel19) (Soria et al. Osimertinib in Untreated EGFR-Mutated Advanced Non-Small-Cell Lung Cancer. N Engl J Med. 2018 378(2):113-125). Unfortunately, while a reasonable number of patients with EGFR-mutant NSCLC respond to EGFR therapy initially, most patients who respond to therapy ultimately develop disease progression after about 9-14 months of treatment due to acquired resistance to osimertinib. The most common mutation leading to resistance includes C797 mutations C797S and C797G (Ercan et al., Clin Can Res 2015 21(17)3913-3923). Cysteine 797 is the site of covalent binding for both rociletinib and osimertinib (Zhou et al., Nature 2009; 462:1070-4).

In a subset of these patients, co-occurring L858R/C797x or ex19del/C797x mutations ("double mutants") are thus emerging as on-target resistance mechanism, underscoring the need for new therapies, particularly in patients with CNS metastases. The exact proportion of cancers that become resistant to first line osimertinib via C797x mutation is still emerging, with the most recent data analyses suggesting mutation frequencies up to 12.5 % (Choudhury et al. Molecular Biomarkers of Disease Outcomes and Mechanisms of Acquired Resistance to First-Line Osimertinib in Advanced EGFR-Mutant Lung Cancers. J Thorac Oncol. 2023 18(4):463-475; Olsen et al. Real-World Clinical Outcomes after Genomic Profiling of Circulating Tumor DNA in Patients with Previously Treated Advanced Non-Small Cell Lung Cancer. Curr Oncol. 2022 29(7):4811-4826; Ramalingam et al., Mechanisms of acquired resistance to first-line osimertinib: Preliminary data from the phase III FLAURA study. Annals Oncol. 2018 29(suppl. 8):viii740; Ramalingam et al. Real-world Landscape of EGFR C797X Mutation as a Resistance Mechanism to Osimertinib in Non-small Cell Lung Cancer. J Thorac Oncol. 2022 17(9, Suppl.):S67-S68; Ramalingam et al. Real-world Landscape of EGFR C797X Mutation as a Resistance Mechanism to Osimertinib in Non-small Cell Lung Cancer. Abstracts, IASLC 2022 World Conference on Lung Cancer, Vienna, Austria. 2022).

Currently, there are no effective therapeutic strategies to overcome the L858R/C797x or ex19del/C797x mutations (double mutations)-mediated EGFR-inhibitor resistance. In theory, first-generation reversible inhibitors can retain potency against C797S double mutant EGFR proteins. However, designing a next generation inhibitor with an improved mutant selectivity profile versus wild-type EGFR could further reduce adverse events related to on-target wild-type EGFR inhibition. In addition, the relative lack of brain exposure to first-generation inhibitors may limit durable clinic responses, particularly in patients with brain metastases (Ballard et al. Preclinical Comparison of Osimertinib with Other EGFR-TKIs in EGFR-Mutant NSCLC Brain Metastases Models, and Early Evidence of Clinical Brain Metastases Activity. Clin Cancer Res. 2016 22(20):5130-5140).

Additional treatment modalities are needed.

### SUMMARY

In one aspect, this disclosure relates to the use of an EGFR inhibitor or a pharmaceutically acceptable salt thereof for treating a cancer having two or more mutations in EGFR, wherein at least one of the mutations is a C797 mutation; preferably the C797S mutation. The EGFR inhibitor disclosed herein is a compound of formula (I): 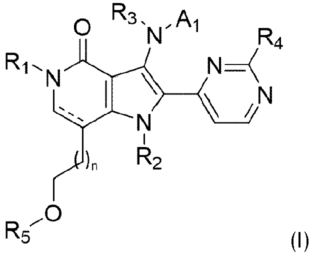 or a pharmaceutically acceptable salt or N-oxide or solvate thereof,
wherein
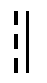 represents a single or double C-C bond,
n is an integer ranging from 0 to 4, preferably n=1,
R₁, R₂, and R₃ are independently H or a (C₁-C₆)alkyl group, preferably H or a methyl group,
R₄ and R₅ are independently a (C₁-C₆)alkyl group, preferably a methyl group,
A₁ is a phenyl group substituted or not substituted by one or more (preferably one or two) substituents selected from the group consisting of a halogen atom, a (C₁-C₆)alkyl group and a (C₁-C₆)alkoxy group.

In another aspect, this disclosure relates to methods of treating a cancer in a subject having two or more mutations in EGFR and in need of such treatment, wherein at least one of the mutations is a C797 mutation; preferably the C797S mutation, the methods include administering an EGFR inhibitor or a pharmaceutically acceptable salt thereof to the subject.

The EGFR inhibitor disclosed herein is a compound of formula (I): 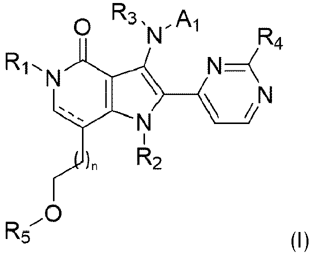 or a pharmaceutically acceptable salt or N-oxide or solvate thereof,
wherein
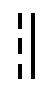 represents a single or double C-C bond,
n is an integer ranging from 0 to 4, preferably n=1,
R₁, R₂, and R₃ are independently H or a (C₁-C₆)alkyl group, preferably H or a methyl group,
R₄ and R₅ are independently a (C₁-C₆)alkyl group, preferably a methyl group,

A₁ is a phenyl group substituted or not substituted by one or more (preferably one or two) substituents selected from the group consisting of a halogen atom, a (C₁-C₆)alkyl group and a (C₁-C₆)alkoxy group.

Preferably, the EGFR inhibitor is a compound of formula (A):

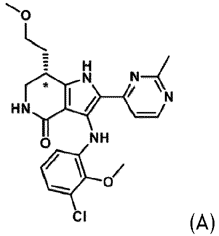

In one embodiment, the mutant EGFR further comprises the mutations L858R or ex19Del.

In one embodiment, EGFR carries two mutations, i.e., EGFR is a double mutant EGFR. Preferably, EGFR has L858R/C797 or ex19del/C797 double mutations, more preferably the L858R/C797S or ex19del/C797S double mutations.

In one embodiment, the patient is subjected to a preliminary test for the presence of two or more EGFR mutations, wherein one of the mutations is a C797 mutation; preferably the C797S mutation. The EGFR inhibitor or pharmaceutically acceptable salt thereof is administered after the presence of two or more EGFR mutations has been confirmed.

In one embodiment, the patient has previously been treated with osimertinib. In particular, the previous treatment with osimertinib failed.

In one embodiment, the cancer is lymphoma, leukaemia, myeloma, Acute myelogenous leukaemia (AML), T-ALL, renal cell carcinoma, colon cancer, colorectal cancer, breast cancer, epithelial squamous cell cancer, melanoma, stomach cancer, brain cancer, lung cancer, pancreatic cancer, cervical cancer, ovarian cancer, liver cancer, bladder cancer, prostate cancer, testicular cancer, thyroid cancer, head and neck cancer, uterine cancer, adenocarcinoma, or adrenal cancer. Preferably, the cancer is a lung cancer, more preferably a non-small cell lung cancer (NSCLC). In particular, the cancer can be a lung cancer, notably a NSCLC, metastasised to the CNS. More particularly, the cancer can be a lung cancer, notably a NSCLC, which has previously been treated with osimertinib and has metastasised to the CNS.

In one embodiment, the EGFR inhibitor or pharmaceutically acceptable salt thereof is combined with one or more other treatment for cancer, preferably at least one additional therapeutic agent. For example, the at least one additional therapeutic agent can be selected in the group consisting of other EGFR-targeted therapeutic agents, such as e.g. osimertinib, gefitinib, erlotinib, afatinib, lapatinib, neratinib, cetuximab, panitumumab, AZD-9291, CL-387785, CO-1686, or WZ4002, other HER2-targeted therapeutic agents, RAS pathway targeted therapeutic agents, PARP inhibitors, other kinase inhibitors (e.g., receptor tyrosine kinase-targeted therapeutic agents, farnesyl transferase inhibitors, signal transduction pathway inhibitors, checkpoint inhibitors, modulators of the apoptosis pathway, e.g., obataclax, cytotoxic chemotherapeutics, angiogenesis-targeted therapies, immune-targeted agents, including immunotherapy, and radiotherapy.

In one embodiment, the EGFR inhibitor or pharmaceutically acceptable salt thereof, and the at least one additional therapeutic agent are administered simultaneously, separately, or sequentially.

In one embodiment, the EGFR inhibitor or pharmaceutically acceptable salt thereof is present in a pharmaceutical composition.

### FIGURE LEGENDS

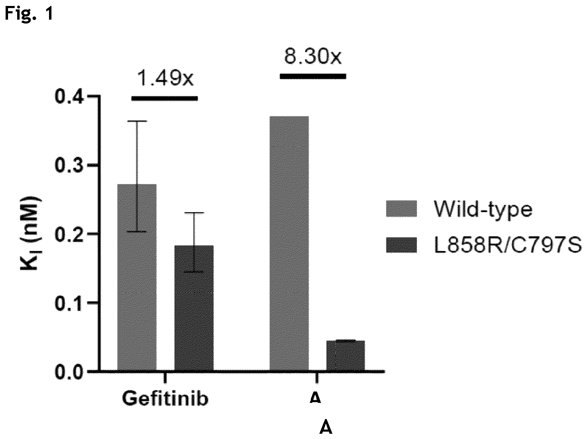

**Fig. 1****: Compound A demonstrates high selectivity in biochemical assays.** Kᵢ was determined for compound A and the benchmark first-generation +GFR inhibitor gefitinib using a Chelation Enhanced Fluorescence (ChEF) biochemical assay with AQT0001 peptide substrate and recombinant EGFR kinase domain proteins (). Data is mean ± standard deviation. Compound A demonstrated more potent inhibition of an EGFR L858R/C797S double mutant than gefitinib. Compound A residence time on the mutant protein was roughly 4.3 hr, relative to 14 min for gefitinib. Compound A selectivity for the L858R/C797S double mutant exceeded that of gefitinib.

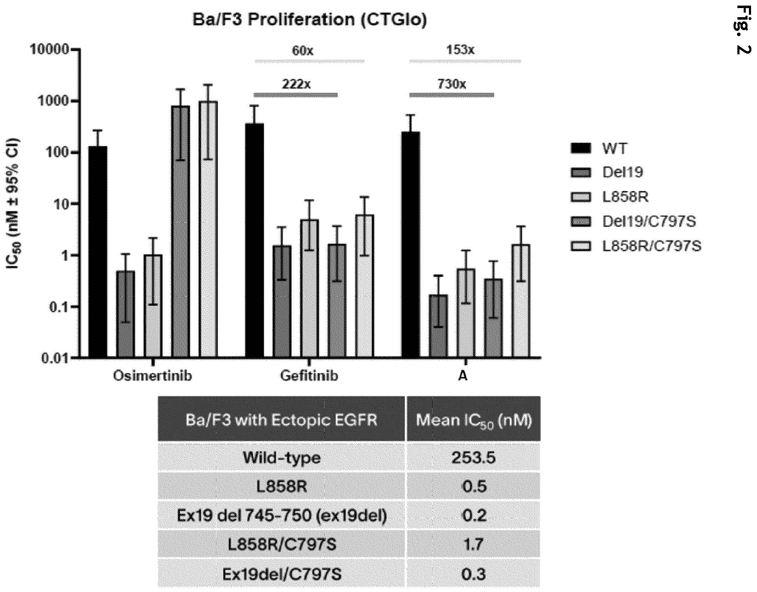

**Fig. 2****: Compound A retains strong potency and selectivity in the presence of C797S.** Left panel: Compound A proliferation inhibition IC50 values for Ba/F3 lines expressing the noted EGFR constructs (CellTiterGlo, 72 hr assay). Right panel: compound A, osimertinib (C297 covalent), and gefitinib (1^{st} gen reversible) mutant selectivity for the noted Ba/F3 cells relative to cells expressing WT EGFR. Compound A retains potent anti proliferative activity and selectivity in the presence of C797S double mutation, and was greater than that observed for the first-generation reversible inhibitor gefitinib (double mutant vs. WT values noted).

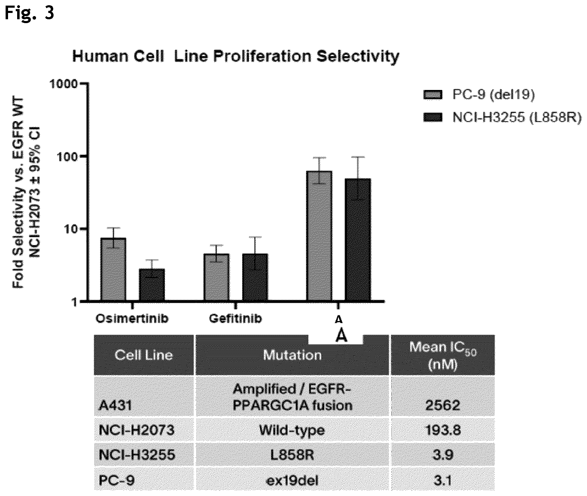

**Fig. 3****: Strong compound A potency and selectivity extends to human cancer cells.** Left panel: Compound A proliferation inhibition IC50 values for the noted human cancer cell lines (CellTiterGlo). Right panel: compound A, osimertinib, and gefitinib mutant cell line selectivity versus the EGFR wild type and EGF-dependent cell line NCI-H2073. compound A selectivity for commonly occurring EGFR mutant cell lines was greater than that observed for either osimertinib or gefitinib.

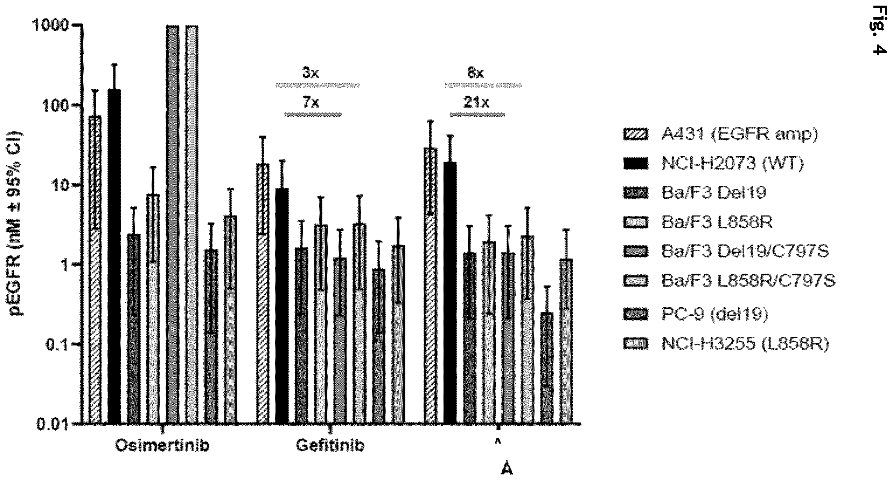

**Fig. 4****: Compound A selectively pEGFR target engagement.** Compound A, osimertinib, and gefitinib IC₅₀ values for pEGFR (phosphorylated EGFR, Tyr1068) inhibition (AlphaLISA). compound A selectivity for all tested EGFR mutant cell lines relative to EGFR wild-type cell line NCI-H2073 cells exceeded that of gefitinib (Ba/F3 double mutant selectivity noted).

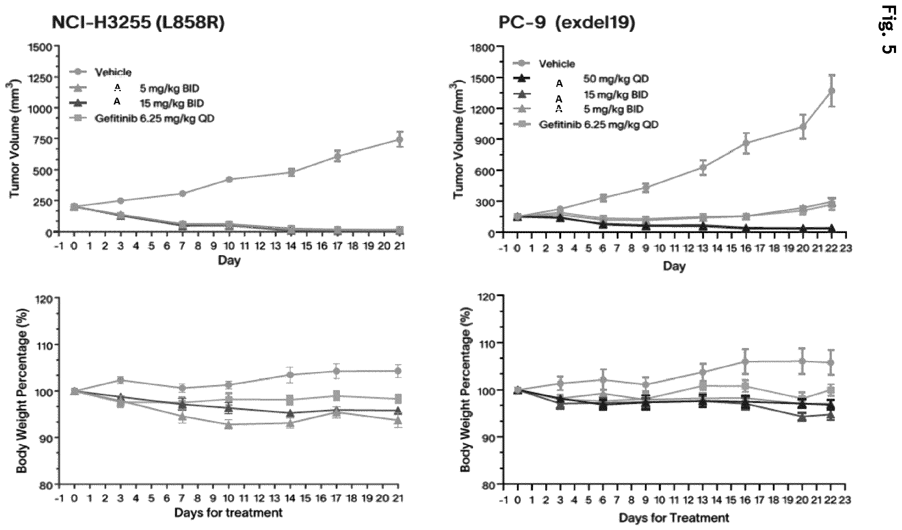

**Fig. 5****: Compound A is efficacious and well tolerated in vivo.** Compound A at 5-15 mg/kg BID provided robust tumour growth inhibition (90 % TGI or greater) in either ex19del or L858R human tumour xenografts in mice. All doses were well tolerated. Antitumour activity was comparable or superior to a published clinically relevant dose of gefitinib.

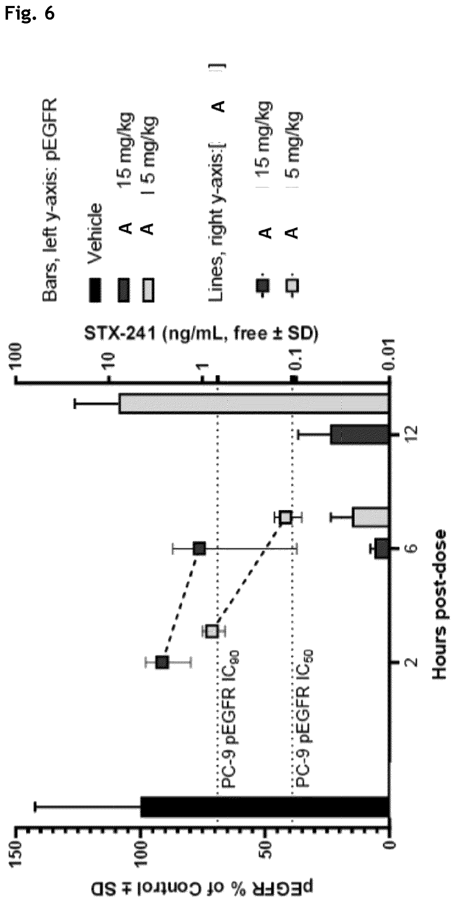

**Fig. 6****: Compound A suppresses pEGFR in PC-9 (ex19del) PK/PD studies.** 5-15 mg/kg (single dose) modulated the pEGFR pharmacodynamic readout, with 15 mg/kg demonstrating > 50% pEGFR inhibition over a 12-hour BID dosing window.

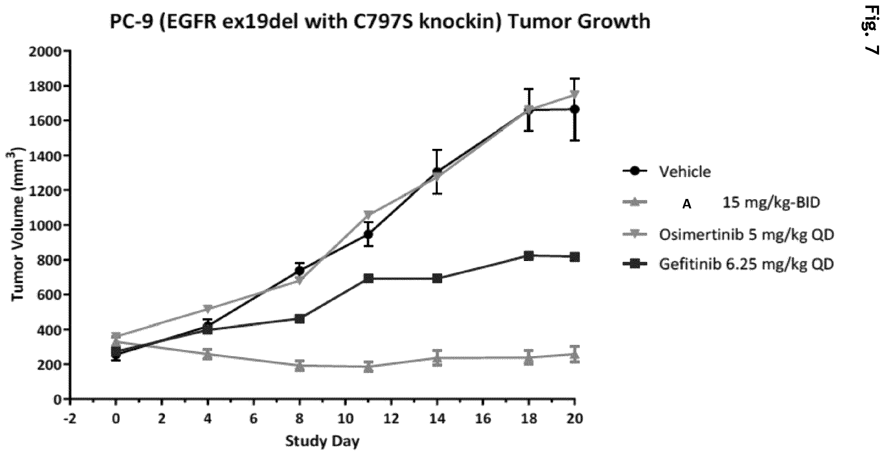

**Fig. 7****: Compound A retains in vivo activity in C797S double mutant xenografts.** A PC-9 derivative with an ex19del/C797S in cis double mutation was engineered by CRISPR knock-in. Compound A at 15 mg/kg BID retained in vivo tumour growth inhibition in the presence of C797S.

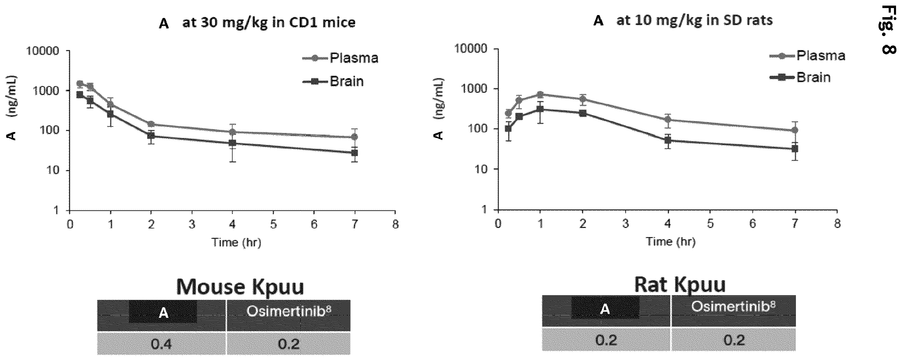

**Fig. 8****: Compound A CNS penetrance is on-par with osimertinib.** Upon oral dosing in blood barrier-intact rodents, Compound A showed good brain distribution with no delay in distribution compared to plasma. Compound A demonstrates high in vitro permeability (CAco-2 ER 1.76 at 1 µM) and low efflux (MDCK-MDR1 ER 0.95, MDCK-BCRP ER 1.17 at 1 µM). Using a rat brain slice method to calculate free fraction in brain, compound A demonstrates brain exposure K_{p,uu} value similar to values obtained for osimertinib using the same method.

### DETAILED DESCRIPTION

### Definitions

To facilitate understanding of the disclosure set forth herein, a number of additional terms are defined below. Generally, the nomenclature used herein and the laboratory procedures in organic chemistry, medicinal chemistry, and pharmacology described herein are those well-known and commonly employed in the art. Unless defined otherwise, all technical and scientific terms used herein generally have the same meaning as commonly understood by one of ordinary skill in the art to which this disclosure belongs. Each of the patents, applications, published applications, and other publications that are mentioned throughout the specification and the attached appendices are incorporated herein by reference in their entireties.

The term "acceptable" with respect to a formulation, composition or ingredient, as used herein, means having no persistent detrimental effect on the general health of the subject being treated.

The term "administer" as used herein refers to the administration of a therapeutically effective dose of a compound or composition disclosed herein to a cell either in cell culture or in a patient (i.e. subject) by any means of administration suitable.

The terms "effective amount" or "therapeutically effective amount," as used herein, refer to a sufficient amount of a chemical entity being administered which will relieve to some extent one or more of the symptoms of the disease or condition being treated. The result includes reduction and/or alleviation of the signs, symptoms, or causes of a disease, or any other desired alteration of a biological system. For example, an "effective amount" for therapeutic uses is the amount of the composition comprising a compound as disclosed herein required to provide a clinically significant decrease in disease symptoms. An appropriate "effective" amount in any individual case is determined using any suitable technique, such as a dose escalation study.

The term "excipient" or "pharmaceutically acceptable excipient" means a pharmaceutically acceptable material, composition, or vehicle, such as a liquid or solid filler, diluent, carrier, solvent, or encapsulating material. In one embodiment, each component is "pharmaceutically acceptable" in the sense of being compatible with the other ingredients of a pharmaceutical formulation, and suitable for use in contact with the tissue or organ of humans and animals without excessive toxicity, irritation, allergic response, immunogenicity, or other problems or complications, commensurate with a reasonable benefit/risk ratio. *See, e.g.,* Remington: The Science and Practice of Pharmacy, 21st ed.; Lippincott Williams & Wilkins: Philadelphia, PA, 2005; Handbook of Pharmaceutical Excipients, 6th ed.; Rowe et al., Eds.; The Pharmaceutical Press and the American Pharmaceutical Association: 2009; Handbook of Pharmaceutical Additives, 3rd ed.; Ash and Ash Eds.; Gower Publishing Company: 2007; Pharmaceutical Preformulation and Formulation, 2nd ed.; Gibson Ed.; CRC Press LLC: Boca Raton, FL, 2009.

The term "mutant epidermal growth factor receptor (EGFR)" as used herein means an EGFR having at least one activating mutation associated with disease. The mutant EGFR can also comprise additional mutations, including drug resistance mutations such as T790M and/or C797S. In particular, a "double mutant EGFR" as used herein means EGFR comprising an activating mutation such as ex19del or L858R and a resistance mutation such as a mutation at T790 (e.g., T790M) or C797 (e.g. C797S). The ex19del refers to small, in frame deletions occurring in exon19 of EGFR (which encodes part of the kinase domain). They primarily occur between codons 746 to 759. This is one of the most prominent EGFR mutations in lung cancer, occurring in about 40% of all EGFR-positive NSCLC patients.

The term "non-small cell lung cancer" as used herein includes adenocarcinomas, squamous cell carcinomas, large cell carcinomas, adenosquamous carcinoma and sarcomatoid carcinoma.

The term "pharmaceutically acceptable salt" refers to a formulation of a compound that does not cause significant irritation to an organism to which it is administered and does not abrogate the biological activity and properties of the compound. In certain instances, pharmaceutically acceptable salts are obtained by reacting a compound described herein, with acids such as hydrochloric acid, hydrobromic acid, sulfuric acid, nitric acid, phosphoric acid, methanesulfonic acid, ethanesulfonic acid, p-toluenesulfonic acid, salicylic acid and the like. In some instances, pharmaceutically acceptable salts are obtained by reacting a compound having acidic group described herein with a base to form a salt such as an ammonium salt, an alkali metal salt, such as a sodium or a potassium salt, an alkaline earth metal salt, such as a calcium or a magnesium salt, a salt of organic bases such as dicyclohexylamine, N-methyl-D-glucamine, tris(hydroxymethyl)methylamine, and salts with amino acids such as arginine, lysine, and the like, or by other methods previously determined. The pharmacologically acceptable salt s not specifically limited as far as it can be used in medicaments. Examples of a salt that the compounds described herein form with a base include the following: salts thereof with inorganic bases such as sodium, potassium, magnesium, calcium, and aluminium; salts thereof with organic bases such as methylamine, ethylamine and ethanolamine; salts thereof with basic amino acids such as lysine and ornithine; and ammonium salt. The salts may be acid addition salts, which are specifically exemplified by acid addition salts with the following: mineral acids such as hydrochloric acid, hydrobromic acid, hydroiodic acid, sulfuric acid, nitric acid, and phosphoric acid; organic acids such as formic acid, acetic acid, propionic acid, oxalic acid, malonic acid, succinic acid, fumaric acid, maleic acid, lactic acid, malic acid, tartaric acid, citric acid, methanesulfonic acid, and ethanesulfonic acid; acidic amino acids such as aspartic acid and glutamic acid.

The term "pharmaceutical composition" refers to a mixture of a compound described herein with other chemical components (referred to collectively herein as "excipients"), such as carriers, stabilisers, diluents, dispersing agents, suspending agents, and/or thickening agents. The pharmaceutical composition facilitates administration of the compound to an organism. Multiple techniques of administering a compound exist in the art including, but not limited to: rectal, oral, intravenous, aerosol, parenteral, ophthalmic, pulmonary, and topical administration.

As used herein, the terms "subject", "individual", or "patient", are used interchangeably, refer to any animal, including mammals and primates (e.g., human), such as mice, rats, other rodents, rabbits, dogs, cats, swine, pig, cow, goat, cattle, sheep, horses, primates, and humans. In some embodiments, the subject is a human. In some embodiments, the subject has experienced and/or exhibited at least one symptom of the disease or disorder to be treated.

As used herein, terms "treat" or "treatment" refer to therapeutic or palliative measures. Beneficial or desired clinical results include, but are not limited to, alleviation, in whole or in part, of symptoms associated with a disease or disorder or condition, diminishment of the extent of disease, stabilised (i.e., not worsening) state of disease, delay or slowing of disease progression, amelioration or palliation of the disease state (e.g., one or more symptoms of the disease), and remission (whether partial or total), whether detectable or undetectable. "Treatment" can also mean prolonging survival as compared to expected survival if not receiving treatment.

### Methods of treatment of cancer carrying EGFR double mutations

Patent application WO 2022/066734A1 discloses new inhibitors of epidermal growth factor receptor (EGFR, ERBB1), notably of mutants of EGFR. This disclosure is based, in part, on the finding that the compounds described herein, e.g., the compounds of formula (I), e.g., compound A, exhibit both (i) relatively high efficacy and selectivity as inhibitors of EGFR double mutants, including L858R/C797x or ex19del/C797x mutations; and (ii) robust CNS penetrance. Indeed, the compounds described herein display strong potency and selectivity against EGFR double mutants, such as EGFR L858R/C797S and ex19del/C797S double mutants, as well as robust CNS penetrance. Mutations in C7997 of EGFR are known to confer resistance to osimertinib (Ercan et al., Clin Can Res 2015 21 (17)3913-3923). The present inhibitors, which target specifically mutant EGFR carrying C797x in combination with other resistance mutations, are therefore particularly useful for treating cancers resistant to osimertinib.

Accordingly, a first aspect of the present disclosure relates to an EGFR inhibitor or a pharmaceutically acceptable salt thereof for the treatment of a cancer having two or more mutations in EGFR, wherein the EGFR inhibitor is a compound of formula (I): 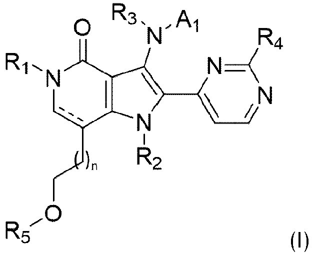 or a pharmaceutically acceptable salt or N-oxide or solvate thereof,
wherein
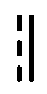 represents a single or double C-C bond,
n is an integer ranging from 0 to 4, preferably n=1,
R₁, R₂, and R₃ are independently H or a (C₁-C₆)alkyl group, preferably H or a methyl group,
R₄ and R₅ are independently a (C₁-C₆)alkyl group, preferably a methyl group,
A₁ is a phenyl group substituted or not substituted by one or more (preferably one or two) substituents selected from the group consisting of a halogen atom, a (C₁-C₆)alkyl group and a (C₁-C₆)alkoxy group.

Preferably, R₁, R₂ and R₃ are H.

Preferably, R₄ and R₅ are methyl.

Advantageously, A₁ is a phenyl group substituted or not substituted by one two substituents selected from the group consisting of a halogen atom and a (C₁-C₆)alkoxy group.

When 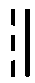 represents a single carbon bond, then the compound of formula (I) may be in the form of the (S) enantiomer, (R) enantiomer, or mixtures thereof, including racemic mixtures thereof. Preferably, then the compound of formula (I) is in the form of the (S) enantiomer.

Preferably, the EGFR inhibitor is a compound of formula (A):

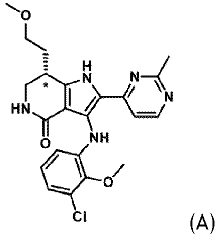

The (*) in the above formula indicates the stereogenic center. Methods for synthesising the present compounds are disclosed in WO 2022/066734A1.

The disclosure also relates to a method of treating a cancer having two or more mutations in EGFR in a patient in need thereof, the method comprising administering to the patient a compound of formula (I) or a pharmaceutically acceptable salt thereof. The disclosure also relates to the use of a compound of formula (I) or a pharmaceutically acceptable salt thereof for making a medicament for the treatment of a cancer having two or more mutations in EGFR.

In an embodiment, one of the EGFR mutations is an EGFR C797 mutation. As used herein, a "C797" or "C797x" mutation refers to any substitution of the cysteine at position 797 in EGFR. Mutations of this residue known to alter EGFR sensitivity to osimertinib include C797S and C797G. Preferably, one of the EGF mutations is the C797S mutation.

More preferably, the mutant EGFR has an EGFR C797 mutation, optionally a C797S mutation, and further comprises EGFR mutations L858R or ex19Del.

In particular, the mutant EGFR is a double-mutant EGFR, i.e., there are two mutations affecting EGFR.

In a preferred embodiment, the mutant EGFR is a L858R/C797 or ex19del/C797 mutant EGFR. Even more preferably, the mutant EGFR is a L858R/C797S or ex19del/C797S mutant EGFR.

In certain embodiments, the cancer is lymphoma, leukaemia, myeloma, Acute myelogenous leukaemia (AML), T-ALL, renal cell carcinoma, colon cancer, colorectal cancer, breast cancer, epithelial squamous cell cancer, melanoma, stomach cancer, brain cancer, lung cancer, pancreatic cancer, cervical cancer, ovarian cancer, liver cancer, bladder cancer, prostate cancer, testicular cancer, thyroid cancer, head and neck cancer, uterine cancer, adenocarcinoma, or adrenal cancer. Preferably, the cancer is a lung cancer. More preferably, the lung cancer is a non-small cell lung cancer (NSCLC). The NSCLC can be an adenocarcinoma, squamous cell carcinoma, large cell carcinoma, adenosquamous carcinoma or a sarcomatoid carcinoma.

It is known that EGFR-mutated NSCLC commonly metastasises to the central nervous system (CNS) metastases, resulting in a poor prognosis and limited treatment options. In particular, the treatment of CNS metastases is particularly challenging given the limited passage of molecules across the blood-CSF barrier and blood-brain barrier (Ahluwalia et al. Epidermal Growth Factor Receptor Tyrosine Kinase Inhibitors for Central Nervous System Metastases from Non-Small Cell Lung Cancer. Oncologist. 2018 23(10):1199-1209).

Surprisingly, the EGFR inhibitors described herein exhibit robust CNS penetrance, making them particularly suitable for treating NSCLC carrying a double-mutant EGFR metastasised to the CNS.

Accordingly, the cancer treated by the methods disclosed herein can be NSCLC having a double-mutant EGFR, e.g., a NSCLC having a L858R/C797 or ex19del/C797 double-mutant EGFR, notably a NCLSC having a L858R/C797S or ex19del/C797S double-mutant EGFR, wherein the NSCLC is metastasised to the CNS.

In some embodiments, the patient is first tested for the presence for the presence of two or more EGFR mutations, notably an EGFR C797 mutation, optionally a C797S mutation, in combination with EGFR mutations L858R or ex19Del. For example, a sample (e.g., a tissue sample, a lung cancer biopsy or a liquid biopsy such as a blood sample or plasma sample for detecting tumour derived DNA and/or circulating tumour cells or circulating exosomes) from the patient is tested for the presence of the mutations. Suitable methods for obtaining tissue samples include tissue biopsy, endobronchial biopsy, transbronchial biopsy, brushing cytology, washing cytology, fine needle aspiration cytology, fluid cytology, or bone biopsy. Testing for EGFR mutations can be done by any suitable analytic technique, including quantitative real-time polymerase chain reaction (PCR), allele-specific PCR, or nucleic acid sequencing. Suitable tests include Therascreen^{®} EGFR RGQ PCR kit (Qiagen), Cobas^{®} EGFR Mutation Test v2 (Roche), FoundationOne CDx^{™} (Foundation Medicine), Oncomine^{™} Dx Target Test (Thermo Fisher Scientific), Guardant360^{™} (Guardant Health), GeneStrat (Biodesix), OncoBEAM^{™} (Sysmex Inostics), ExoDx^{®} Lung (T790M) (Exosome Diagnostics), and Biocept liquid biopsy. If the mutations are present, the patient is administered a compound of formula (I) or a pharmaceutically acceptable salt thereof as described herein.

In another aspect, the patient has previously received one chemotherapy regimen. Preferably, the cancer has failed at least one prior chemotherapy regimen. For example, the cancer is a cancer that has failed at least one prior osimertinib regimen. Indeed, limited strategies are available at disease progression on osimertinib for patients with mutant-EGFR cancer, in particular NSCLC. The emergence of the on-target EGFR C797S mutation has been described as one of the most common mechanisms of resistance to osimertinib exposure. Remarkably, by studying the molecular profile at progression, it has been reported that the presence of the EGFR-sensitising mutation, C797S resulted in resistance to Osimertinib. Thus, the efficacy of any compound with tumours exhibiting C797S mutation represent excellent candidate for the treatment of patients wherein Osimertinib failed.

In one embodiment, the EGFR inhibitor disclosed herein is thus for the treatment of cancer, wherein the treatment comprises administering the EGFR inhibitor to a patient who has previously been treated with osimertinib. The present disclosure also relates to a method of treating a cancer in a patient in need thereof, wherein the method comprises administering the EGFR inhibitor to the patient and wherein the patient has previously been treated with osimertinib. The present disclosure also relates to the use of an EGFR inhibitor for making a medicament for the treatment of cancer, wherein the treatment comprises administering the EGFR inhibitor to a patient who has previously been treated with osimertinib. Preferably, the cancer has failed the prior round of osimertinib treatment. The cancer can be notably a lung cancer, e.g., a non-small cell lung cancer (NSCLC). In particular, the cancer can be a lung cancer, notably a NSCLC, metastasised to the CNS. More particularly, the cancer can be a lung cancer, notably a NSCLC, which has a previously been treated with osimertinib and has metastasised to the CNS.

The treatments with the EGFR inhibitor disclosed herein can also be combined with one or more other treatments for cancer.

The treatment for cancer can for example be surgery, chemotherapy, adjuvant therapy, radiation therapy, other targeted therapy or a combination thereof.

For example, the compound of Formula (I) (or a pharmaceutically acceptable salt thereof) can be combined with a therapeutically effective amount of at least one additional therapeutic agent selected from one or more additional therapies or therapeutic (e.g., chemotherapeutic) agents.

The compound of Formula (I) (or a pharmaceutically acceptable salt thereof) and the at least one additional therapeutic agent can notably be administered simultaneously, separately, or sequentially.

Non-limiting examples of additional therapeutic agents include: other EGFR-targeted therapeutic agents (i.e., a first or second EGFR inhibitor), such as e.g. osimertinib, gefitinib, erlotinib, afatinib, lapatinib, neratinib, cetuximab, panitumumab, AZD-9291, CL-387785, CO-1686, or WZ4002, other HER2-targeted therapeutic agents (i.e., a first or second HER2 inhibitor), RAS pathway targeted therapeutic agents, PARP inhibitors, other kinase inhibitors (e.g., receptor tyrosine kinase-targeted therapeutic agents (e.g., Trk inhibitors or multi-kinase inhibitors)), farnesyl transferase inhibitors, signal transduction pathway inhibitors, checkpoint inhibitors, modulators of the apoptosis pathway (e.g., obataclax); cytotoxic chemotherapeutics, angiogenesis-targeted therapies, immune-targeted agents, including immunotherapy, and radiotherapy.

In some embodiments, the additional therapeutic agent is selected from the group consisting of osimertinib, gefitinib, erlotinib, afatinib, lapatinib, neratinib, AZD-9291, CL-387785, CO-1686, or WZ4002.

### Pharmaceutical compositions

As will be easily realised, the compounds of formula (I) or pharmaceutically acceptable salts thereof are usually administered to the patients in pharmaceutical compositions.

Therefore, the present description also provides a pharmaceutical composition comprising a compound of formula (I) such as defined above and at least one pharmaceutically acceptable excipient, for the medical uses disclosed herein.

The form of the pharmaceutical compositions, the route of administration, the dosage and the regimen naturally depend upon the condition to be treated, the severity of the illness, the age, weight, and sex of the patient, etc.

The pharmaceutical compositions of the invention can be formulated for a topical, oral, intranasal, parenteral, intraocular, intravenous, intramuscular or subcutaneous administration and the like.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses used for the administration can be adapted as a function of various parameters, and in particular as a function of the mode of administration used, of the relevant pathology, or alternatively of the desired duration of treatment.

The active ingredient can be administered in unit forms of administration, in a mixture with conventional pharmaceutical carriers, to animals or to human beings. Suitable unit forms of administration comprise forms via oral route, forms for sublingual or buccal administration, forms for administration via parenteral route (subcutaneous, intradermal, intramuscular or intravenous), forms for topical administration (on the skin and mucosa, including intranasal and intraocular administration) and forms for rectal administration.

Such compositions may be in the form of a solid, liquid, emulsion, lotion or cream. As solid compositions, for oral administration, use can be made of tablets, pills, powders (hard or soft gelatine capsules) or granules. In these compositions, the active ingredient of the disclosure is mixed with one or more inert diluents such as starch, cellulose, sucrose, lactose or silica, in a stream of argon. These compositions may also comprise substances other than diluents, for example one or more lubricants such as magnesium stearate or talc, a colouring agent, a coating (coated tablets) or a varnish.

As liquid compositions for oral administration, use can be made of solutions, suspensions, emulsions, syrups and elixirs that are pharmaceutically acceptable and contain inert diluents such as water, ethanol, glycerol, vegetable oils or paraffin oil. These compositions may comprise substances other than diluents; for example, wetting, sweetening, thickening, flavouring or stabilising products.

The sterile compositions for parenteral administration may preferably be aqueous or non-aqueous solutions, suspensions or emulsions. As solvent or vehicle, use can be made of water, propyleneglycol, a polyethyleneglycol, vegetable oils, in particular olive oil, injectable organic esters e.g., ethyl oleate or other suitable organic solvents. These compositions may also contain adjuvants, in particular wetting, isotonic, emulsifying, dispersing and stabilising agents. Sterilisation can be performed in several manners, for example by sanitising filtration, by incorporating sterilising agents into the composition, by radiation or by heating. They can also be prepared in the form of solid sterile compositions which can be dissolved at the time of use in sterile water or any other injectable sterile medium.

The compositions for rectal administration are suppositories or rectal capsules which, in addition to the active ingredient, contain excipients such as cocoa butter, semi-synthetic glycerides or polyethyleneglycols.

The compositions for topical administration may for example be creams, lotions, eye drops, mouthwash, nasal drops or sprays.

The doses are dependent on the desired effect, on the length of treatment and the route of administration used. In general, the physician will determine the suitable dosage in relation to the age, weight and all other factors particular to the patient to be treated.

The pharmaceutical compositions according to the present disclosure can further comprise an additional therapeutic agent. In particular, the pharmaceutical compositions disclosed herein can comprise one or more of: other EGFR-targeted therapeutic agents (i.e., a first or second EGFR inhibitor), such as e.g. osimertinib, gefitinib, erlotinib, afatinib, lapatinib, neratinib, cetuximab, panitumumab, AZD-9291, CL-387785, CO-1686, or WZ4002, other HER2-targeted therapeutic agents (i.e., a first or second HER2 inhibitor), RAS pathway targeted therapeutic agents, PARP inhibitors, other kinase inhibitors (e.g., receptor tyrosine kinase-targeted therapeutic agents (e.g., Trk inhibitors or multi-kinase inhibitors)), farnesyl transferase inhibitors, signal transduction pathway inhibitors, checkpoint inhibitors, modulators of the apoptosis pathway (e.g., obataclax); cytotoxic chemotherapeutics, angiogenesis-targeted therapies, immune-targeted agents, including immunotherapy, and radiotherapy.

In some embodiments, the additional therapeutic agent is selected from the group consisting of osimertinib, gefitinib, erlotinib, afatinib, lapatinib, neratinib, AZD-9291, CL-387785, CO-1686, or WZ4002.

### Dosages

The dosages may be varied depending on the requirement of the patient, the severity of the condition being treated and the particular compound being employed. Determination of the proper dosage for a particular situation can be determined by one skilled in the medical arts. The total daily dosage may be divided and administered in portions throughout the day or by means providing continuous delivery.

In some embodiments, the compounds described herein are administered at a dosage of from about 0.001 mg/Kg to about 500 mg/Kg (e.g., from about 0.001 mg/Kg to about 200 mg/Kg; from about 0.01 mg/Kg to about 200 mg/Kg; from about 0.01 mg/Kg to about 150 mg/Kg; from about 0.01 mg/Kg to about 100 mg/Kg; from about 0.01 mg/Kg to about 50 mg/Kg; from about 0.01 mg/Kg to about 10 mg/Kg; from about 0.01 mg/Kg to about 5 mg/Kg; from about 0.01 mg/Kg to about 1 mg/Kg; from about 0.01 mg/Kg to about 0.5 mg/Kg; from about 0.01 mg/Kg to about 0.1 mg/Kg; from about 0. 1 mg/Kg to about 200 mg/Kg; from about 0. 1 mg/Kg to about 150 mg/Kg; from about 0. 1 mg/Kg to about 100 mg/Kg; from about 0.1 mg/Kg to about 50 mg/Kg; from about 0. 1 mg/Kg to about 10 mg/Kg; from about 0. 1 mg/Kg to about 5 mg/Kg; from about 0. 1 mg/Kg to about 1 mg/Kg; from about 0. 1 mg/Kg to about 0.5 mg/Kg).

### Regimens

The foregoing dosages can be administered on a daily basis (e.g., as a single dose or as two or more divided doses) or non-daily basis (e.g., every other day, every two days, every three days, once weekly, twice weeks, once every two weeks, once a month).

In some embodiments, the period of administration of a compound described herein is for 1 day, 2 days, 3 days, 4 days, 5 days, 6 days, 7 days, 8 days, 9 days, 10 days, 1 1 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 1 1 months, 12 months, or more. In a further embodiment, a period of during which administration is stopped is for 1 day, 2 days, 3 days, 4 days, 5 days, 6 days, 7 days, 8 days, 9 days, 10 days, 1 1 days, 12 days, 13 days, 14 days, 3 weeks, 4 weeks, 5 weeks, 6 weeks, 7 weeks, 8 weeks, 9 weeks, 10 weeks, 1 1 weeks, 12 weeks, 4 months, 5 months, 6 months, 7 months, 8 months, 9 months, 10 months, 1 1 months, 12 months, or more. In an embodiment, a therapeutic compound is administered to an individual for a period of time followed by a separate period of time. In another embodiment, a therapeutic compound is administered for a first period and a second period following the first period, with administration stopped during the second period, followed by a third period where administration of the therapeutic compound is started and then a fourth period following the third period where administration is stopped. In an aspect of this embodiment, the period of administration of a therapeutic compound followed by a period where administration is stopped is repeated for a determined or undetermined period of time. In a further embodiment, a period of administration is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more. In a further embodiment, a period of during which administration is stopped is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more.

Hereinbelow, the present invention is explained in detail in view of the examples. However, the following examples are given only for exemplification of the present invention, and it is evident that the present invention is not limited to the following examples.

### EXAMPLES

In order to characterise compound A in vitro and in vivo potency and selectivity for C797S-active double-mutant inhibitor vs. EGFR wild type, compound A was assessed in vitro and in vivo across a variety of engineered or endogenously occurring single (ex19del or L858R) or double (ex19del/C797S or L858R/C797S) mutant systems. In addition, the CNS exposure of compound A was also characterised using a gold-standard rat brain slice method to assess free exposure in brain tissue.

### Materials and methods:

Compound A is an EGFR inhibitor of formula I(a). Compound A was tested across a panel of in vitro biochemical, cell signalling, and proliferation assays for potency against EGFR L858R and ex19del single mutants and/or the corresponding C797S double mutants. Additionally, compound A was tested for in vivo activity in mice bearing human NSCLC cell line xenografts NCI-H3255 (L858R) and PC-9 (ex19del), and in a PC-9-derived ex19del/C797S double mutant knock-in xenograft. Free CNS penetration (K_{p,uu}) was determined in nontumour bearing mice and rats. Osimertinib and/or gefitinib were used as benchmark molecules across assays.

### Results:

Compound A, an ATP-competitive reversible EGFR inhibitor representing novel chemical matter, demonstrated potent and selective inhibition of recombinant EGFR L858R/C797S mutant EGFR protein relative to wild-type, with increased mutant residence time relative to gefitinib, an approved reversible EGFR inhibitor. Potent (high picomolar to low nanomolar) inhibition of L858R, ex19del, L858R/C797S and ex19del/C797S mutants was observed in proliferation assays using engineered Ba/F3 and human NSCLC cell lines. >150x selectivity for all tested mutants relative to wild-type EGFR was demonstrated in engineered Ba/F3 cells and human cancer cells; this level of selectivity was in excess of that observed for gefitinib. Strong potency and double mutant selectivity was also observed for compound A in pharmacodynamic assays measuring EGFR pathway activation (pEGFR). compound A was well tolerated at doses of 15 mg/kg BID or 50 mg/kg QD in mice, where regression of EGFR single mutant xenografts was observed, concomitant with EGFR pathway suppression. Notably, using an isogenic pair of PC-9 (EGFR ex19del) NSCLC xenografts that differ only by the presence of a C797S mutation, compound A demonstrates no drop-off in antitumour activity in the presence of C797S. In mouse rat pharmacokinetic studies measuring free CNS penetration, K_{p,uu} measurements were comparable to Osimertinib, run in parallel as a benchmark.

### Conclusions

Compound A demonstrates strong biochemical inhibition of EGFR double mutant kinase activity and a wider selectivity that then first-generation reversible compound gefitinib.

Compound A is potent and selective for C797S double mutants relative to wild-type EGFR across proliferation and target engagement assays. The observed in vitro potency and selectivity exceeds that of gefitinib in double mutant cell lines.
compound A displays strong in vivo antitumour activity across tested single and double mutant CDX models and retains in vivo activity in the presence of C797S mutation.

With low efflux and good unbound exposure in brain tissue, compound A demonstrates brain exposure on-par with that osimertinib, providing potential to address CNS metastases.

Compound A preclinical data indicates that it is a potential best-in-class 4^{th} generation double-mutant EGFR inhibitor.

## Claims

1. An EGFR inhibitor or a pharmaceutically acceptable salt thereof for use in treating a cancer having two or more mutations in EGFR,
wherein at least one of the mutations is a C797 mutation; preferably the C797S mutation, and
wherein the EGFR inhibitor is a compound of formula (I): 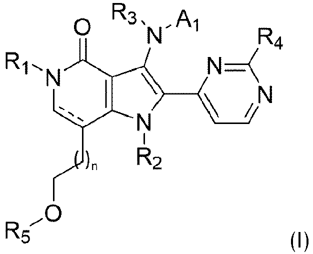 or a pharmaceutically acceptable salt or N-oxide or solvate thereof,
wherein
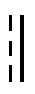 represents a single or double C-C bond,
n is an integer ranging from 0 to 4, preferably n=1,
R₁, R₂, and R₃ are independently H or a (C₁-C₆)alkyl group, preferably H or a methyl group,
R₄ and R₅ are independently a (C₁-C₆)alkyl group, preferably a methyl group,
A₁ is a phenyl group substituted or not substituted by one or more (preferably one or two) substituents selected from the group consisting of a halogen atom, a (C₁-C₆)alkyl group and a (C₁-C₆)alkoxy group.

2. The EGFR inhibitor or a pharmaceutically acceptable salt thereof for the use of claim 1, wherein the EGFR inhibitor is a compound of formula (A):

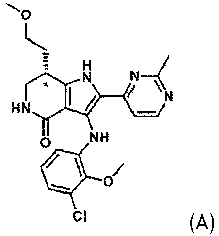

3. The EGFR inhibitor or a pharmaceutically acceptable salt thereof for the use of any one of claims 1 or 2, wherein the mutant EGFR further comprises the mutations L858R or ex19Del.

4. The EGFR inhibitor or a pharmaceutically acceptable salt thereof for the use of claim 3, wherein EGFR carries two mutations.

5. The EGFR inhibitor or a pharmaceutically acceptable salt thereof for the use of claim 4, wherein EGFR has L858R/C797 or ex19del/C797 double mutations, preferably the L858R/C797S or ex19del/C797S double mutations.

6. The EGFR inhibitor or a pharmaceutically acceptable salt thereof for the use of any one of claims 1 to 5, further comprising a prior step of testing the patient for the presence of two or more EGFR mutations, wherein one of the mutations is a C797 mutation; preferably the C797S mutation.

7. The EGFR inhibitor or a pharmaceutically acceptable salt thereof for the use of any one of claims 1 to 6, the use comprising administering the EGFR inhibitor to a patient previously treated with osimertinib.

8. The EGFR inhibitor or a pharmaceutically acceptable salt thereof for the use of claim 7, wherein the previous treatment with osimertinib failed.

9. The EGFR inhibitor or a pharmaceutically acceptable salt thereof for the use of any one of claims 1 to 8, wherein the cancer is lymphoma, leukaemia, myeloma, Acute myelogenous leukaemia (AML), T-ALL, renal cell carcinoma, colon cancer, colorectal cancer, breast cancer, epithelial squamous cell cancer, melanoma, stomach cancer, brain cancer, lung cancer, pancreatic cancer, cervical cancer, ovarian cancer, liver cancer, bladder cancer, prostate cancer, testicular cancer, thyroid cancer, head and neck cancer, uterine cancer, adenocarcinoma, or adrenal cancer.

10. The EGFR inhibitor or a pharmaceutically acceptable salt thereof for the use of claim 9, wherein the cancer is a lung cancer, preferably a non-small cell lung cancer (NSCLC).

11. The EGFR inhibitor or a pharmaceutically acceptable salt thereof for the use of claim 10, wherein the NSCLC is metastasised to the CNS.

12. The EGFR inhibitor or a pharmaceutically acceptable salt thereof for the use of any one of claims 1 to 11, wherein the use comprises the EGFR inhibitor or pharmaceutically acceptable salt thereof is combined with one or more other treatment for cancer, preferably at least one additional therapeutic agent.

13. The EGFR inhibitor or a pharmaceutically acceptable salt thereof for the use of claim 12, wherein the at least one additional therapeutic agent is selected in the group consisting of other EGFR-targeted therapeutic agents, such as e.g. osimertinib, gefitinib, erlotinib, afatinib, lapatinib, neratinib, cetuximab, panitumumab, AZD-9291, CL-387785, CO-1686, or WZ4002, other HER2-targeted therapeutic agents, RAS pathway targeted therapeutic agents, PARP inhibitors, other kinase inhibitors (e.g., receptor tyrosine kinase-targeted therapeutic agents, farnesyl transferase inhibitors, signal transduction pathway inhibitors, checkpoint inhibitors, modulators of the apoptosis pathway ,e.g., obataclax, cytotoxic chemotherapeutics, angiogenesis-targeted therapies, immune-targeted agents, including immunotherapy, and radiotherapy.

14. The EGFR inhibitor or a pharmaceutically acceptable salt thereof for the use of any one of claims 12 or 13, wherein the EGFR inhibitor or pharmaceutically acceptable salt thereof, and the at least one additional therapeutic agent are administered simultaneously, separately, or sequentially.

15. The EGFR inhibitor or a pharmaceutically acceptable salt thereof for the use of any one of claims 1 to 12, wherein the EGFR inhibitor or pharmaceutically acceptable salt thereof is present in a pharmaceutical composition.
